# EUROPEAN PATENT APPLICATION

(11) **EP 1 875 914 A1**
(43) Date of publication of application: **09.01.2008**
(21) Application number: 06255751.7
(22) Date of filing: 09.11.2006
(51) Int. Cl.: A61K 31/4985, A61K 9/08, A61K 47/38, A61K 47/34, A61K 47/32, A61K 47/10

(54) **Solid compositions comprising tadalafil and at least one carrier**

(30) Priority: 07.07.2006 US 819215 P
(71) Applicant: TEVA PHARMACEUTICAL INDUSTRIES LTD., 49131 Petah Tiqva (IL)
(72) Inventor: Zalit, IIan, Rosh Haain (IL); Pal, Boaz, Tel-Aviv (IL)
(74) Representative: Wong, Kit Yee

(57) **Abstract**

The invention relates to solid composites comprising tadalafil, methods for their preparation, and pharmaceutical compositions comprising the solid composites.

## Description

### FIELD OF THE INVENTION

This invention relates to oral pharmaceutical compositions suitable for making pharmaceutical formulations for oral administration that provide for the rapid dissolution of the phosphodiesterase 5 inhibitor tadalafil. In particular, the pharmaceutical compositions comprise solid composites of tadalafil exhibiting high solubility and rate of dissolution. The invention further relates to methods of preparing these pharmaceutical formulations and the use of such pharmaceutical formulations for treating diseases associated with PDE5 inhibitors.

### BACKGROUND OF THE INVENTION

A wide variety of biological processes, including cardiac muscle contraction, regulation of blood flow, neural transmission, glandular secretion, cell differentiation and gene expression are reportedly affected by steady state levels of the cyclic nucleotide biological second messengers cAMP and cGMP. Intracellular receptors for these molecules apparently include cyclic nucleotide dependent protein kinases (PGK), cyclic nucleotide-gated channels, and class I phosphodiesterases (PDEs). PDEs are a large family of proteins, which were understood to be reported by Sutherland and co-workers (Rall & Sutherland 1958, Butcher & Sutherland 1962). The family of cyclic nucleotide phosphodiesterases appears to catalyze the hydrolysis of 3', 5'-cyclic nucleotides to the corresponding 5' monophosphates.

Tadalafil, the active ingredient in Cialis®, has been used for the treatment of male erectile dysfunction (prescribing information). The prescribing information for Cialis® describes this product as film-coated, almond-shaped tablets for oral administration, containing tadalafil and the following inactive ingredients: croscarmellose sodium, hydroxypropyl cellulose, hypromellose, iron oxide, lactose monohydrate, magnesium stearate, microcrystalline cellulose, sodium lauryl sulfate, talc, titanium dioxide, and triacetin. See http://pi.lilly.com/us/cialis-pi.pdf.

Tadalafil apparently has the chemical name (6R-trans)-6-(1,3-benzodioxol-5-yl)-2,3,6,7,12,12a-hexahydro-2-methyl-pyrazino[1',2':1,6]pyrido[3,4-b]indole-1,4-dione. The reported structure of tadalafil is shown below. (CAS# 171596-29-5).

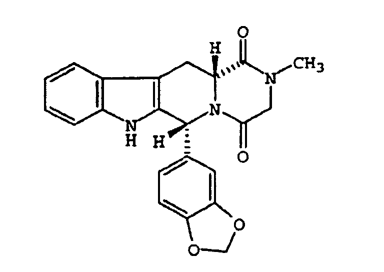

Tadalafil is a solid that is understood to be practically insoluble in water and only very slightly soluble in some organic solvents, such as methanol, ethanol, and acetone. For example, United States Patent No. 6,841,167 reports that tadalafil has a water solubility of about 2 µg per milliliter of water at 25°C.

Compounds having a low water solubility typically can have a low rate of dissolution and low bioavailability. See, e.g., Ansel, et al., Pharmaceutical Dosage Forms and Delivery Methods (6^{th} ed., 1995), p. 105, 108.

Different techniques have been applied in an attempt to overcome the apparent poor solubility of tadalafil. Published International Patent Application WO 01/08686 seems to disclose a pharmaceutical formulation comprising tadalafil in "free drug" form in admixture with a diluent, lubricant, a hydrophilic binder, and a disintegrant, although there is perhaps confusion as to the meanings of the terms used.

Another technique involves preparing formulations using "co-precipitates" of tadalafil, wherein an "intimate mixture" of tadalafil and a carrier in a non aqueous water miscible solvent and optionally water, possibly in solution, are co-precipitated from the "intimate mixture" using an aqueous "co-precipitation medium" in which the carrier is substantially insoluble. See U.S. Patent No. 5,985,326, where once again some clarity may be absent. The '326 patent is disparaging regarding so-called solvent based processes.

Soft gel capsules containing tadalafil suspended in a pharmaceutically acceptable solvent have also apparently been developed in an attempt to prepare formulations of tadalafil with supposedly improved bioavailability.

U.S. Patent No. 6,821,975 is listed in the FDA's "Orange Book" for the Cialis® product, and is assigned on its face to the company that markets Cialis®. This patent appears to be directed to a "free drug particulate form" of tadalafil "comprising particles of the compound wherein at least 90% of the particles have a particle size of less than about 40 microns." Apparently, preferably at least 90% of the particles have a particle size of less than 10 microns.

However, it is believed that even with application of such techniques, only a fraction of the tadalafil present reaches the bloodstream upon administration of the formulations.

There is a continuing need in the art for pharmaceutical formulations of tadalafil with improved rates of dissolution and improved bioavailability. The instant invention provides pharmaceutical formulations or compositions of tadalafil with significantly improved solubility and rates of dissolution as compared with both Cialis® and very fine particle sized free drug tadalafil.

### SUMMARY OF THE INVENTION

In one aspect, the present invention relates to a solid composite comprising tadalafil and at least one carrier, wherein at least 85% by weight (wt%) of the tadalafil is in intimate association with the at least one carrier (i.e. at least 85% by weight of the tadalafil is in a single phase with the at least one carrier). Preferably, at least 85 wt% of the tadalafil is not in crystalline form. It is preferred that the solid composites are solid solutions.

It is further preferred that at least 80 wt% of the tadalafil dissolves out of the solid solution within about 10 minutes when a sample of solid solution containing about 20 mg of tadalafil is tested in 1000 mL of 0.15 wt% sodium lauryl sulfate and 6g/L NaH₂PO₄, having a pH of about 4.5 at 37°C, with paddles rotating at a speed of 50 rpm. Preferably, at least 60 wt%, more preferably at least 70 wt%, of the tadalafil dissolves out of the solid solution within about 5 minutes under these conditions.

The solid composites may be included in a pharmaceutical composition that also include tadalafil in free drug form and having a particle size distribution such that the d(0.9) is greater than about 40 µm. Preferably, in such pharmaceutical compositions, 50 wt% of the tadalafil of the pharmaceutical composition dissolves within about 5 minutes and 70 wt% of the tadalafil of the pharmaceutical composition dissolves within about 10 minutes when a sample of the pharmaceutical composition containing about 20 mg tadalafil is tested in 1000 ml of an 0.15 wt% sodium lauryl sulfate and 6g/L NaH₂PO₄, having a pH of about 4.5 at 37°C with paddles rotating at a speed of 50 rpm.

It is further preferred that substantially all of the tadalafil is in the single phase. It is also preferred that the carrier is a hydrophilic polymer. Preferably, the ratio of tadalafil to polymer is from about 1:0.5 to about 1:20, more preferably the ratio is from about 1:1 to about 1:10, even more preferably the ratio is from about 1:3 to about 1:6, and more preferably the ratio is about 1:5 by weight. Preferably, the carrier is povidone, hydroxypropyl methylcellulose, or polyethylene glycol. More preferably, the carrier is povidone (also known as polyvinylpyrrolidone).

In another aspect, the invention relates to a solid composite including tadalafil and at least one carrier wherein at least about 85 wt% of the tadalafil is in solid solution in the at least one carrier. Preferably, at least 80 wt% of the tadalafil dissolves within about 10 minutes when a sample of the solid composite containing about 20 mg of tadalafil is tested in 1000 mL of 0.15 wt% sodium lauryl sulfate and 6g/L NaH₂PO₄, having a pH of about 4.5 at 37°C, with paddles rotating at a speed of 50 rpm.

The solid composites can be included in pharmaceutical compositions that also include tadalafil in free drug form and having a particle size distribution such that the d(0.9) value is greater than about 40 µm. Preferably, in such pharmaceutical compositions, 50 wt% of the tadalafil of the pharmaceutical composition dissolves within about 5 minutes and 70 wt% of the tadalafil of the pharmaceutical composition dissolves within about 10 minutes when a sample of the pharmaceutical composition containing about 20 mg of tadalafil is tested in 1000 mL of 0.15 wt% sodium lauryl sulfate and 6g/L NaH₂PO₄, having a pH of about 4.5 at 37°C, with paddles rotating at a speed of 50 rpm.

Preferably, in the solid composites, substantially all of the tadalafil is in solid solution in the at least one carrier. It is also preferred that the at least one carrier is a hydrophilic polymer. Preferably, the at least one carrier is present in an amount such that the ratio of the tadalafil to the at least one carrier is from about 1:3 to about 1:6 by weight. Preferably, the at least one carrier is povidone, hydroxypropyl methylcellulose, or polyethylene glycol. Povidone is especially preferred.

In another aspect, the invention relates to a method of preparing a solid composite including tadalafil and at least one carrier. The method includes the steps of: a) combining tadalafil, at least one carrier and at least one solvent to form a solution; and b) removing the solvent from the solution to obtain the solid composite.

Preferably, in the method, the solid composite is a solid solution. It is further preferred that the carrier is povidone, hydroxypropyl methylcellulose, or polyethylene glycol. It is also preferred that the solvent is a lower aliphatic alcohol or C₃ - C₈ ketone. Preferably, the solvent is removed by evaporation. It is preferred that the solvent removal is effected with a fluidized bed dryer, preferably by spraying the solution into an initially empty fluidized bed dryer. The solution of step a) can be sprayed into an initially empty fluidized bed drier to remove the solvent. Preferably, the solution is sprayed onto at least one pharmaceutically acceptable excipient in the fluidized bed dryer. In another aspect, the solvent is removed by spray drying.

In yet another aspect, the invention relates to a solid composite including tadalafil that, when tested in an aqueous solution having a pH between about 4.5 and 7.0, at least 80 wt% of the tadalafil is released from the solid composite or a pharmaceutical formulation including the composite within about 10 minutes. Preferably, at least 60 wt% of the tadalafil is released within about 5 minutes. Preferably, the solid composite includes at least one carrier.

In yet another aspect, the invention relates to a solid composite including tadalafil that, when tested in an aqueous solution having a pH between about 4.5 and 7.0, at least 80 wt% of the tadalafil dissolves within about 10 minutes. Preferably, at least 60 wt% of the tadalafil dissolves within about 5 minutes. Preferably, the solid composite includes at least one carrier.

In another aspect, the invention relates to a solid composite including about 20 mg of tadalafil wherein at least 80 wt% of the tadalafil dissolves within about 10 minutes when tested in 1000 mL of 0.15 wt% sodium lauryl sulfate and 6g/L NaH₂PO₄, having a pH of about 4.5 at 37°C, with paddles rotating at a speed of 50 rpm. Preferably, at least 60 wt% of the tadalafil dissolves within about 5 minutes. Preferably, the solid composite includes at least one carrier.

In a further aspect, the invention relates to a tadalafil solid composite having a higher solubility (or at least a higher solubility in water-based media) as compared with micronized free drug forms of tadalafil.

In another aspect, the invention relates to a tadalafil solid solution having a higher solubility (or at least a higher solubility in water-based media) as compared with a free drug form of tadalafil having a particle size distribution such that the d₉₀ value is about 4 µm.

In another aspect, the invention relates to a pharmaceutical composition including tadalafil in a non-crystalline form. Preferably, at least about 85 wt% of the tadalafil is in non-crystalline form.

In yet another aspect, the invention relates to a pharmaceutical composition including tadalafil in an amorphous form.

In another aspect, the invention relates to a solid composite including tadalafil and polyvinylpyrrolidone wherein the tadalafil and polyvinylpyrrolidone are in intimate association that is formed by evaporating the solvent from a solution of tadalafil and polyvinylpyrrolidone. Preferably, the tadalafil and polyvinylpyrrolidone are present in a ratio of about 1:5 by weight.

In another aspect, the invention relates to a pharmaceutical composition of tadalafil including a) tadalafil in amorphous form in intimate association with at least one carrier, in combination with b) tadalafil in free drug form having a particle size distribution such the d(0.9) value is greater than about 40 µm. Preferably, parts a) and b) are present in such proportion that, when a sample of the composition containing about 20 mg of tadalafil is tested, 50 wt% of the tadalafil is released from the composition within about 5 minutes and 70 wt% of the tadalafil is released from the composition within about 10 minutes when tested in 1000 mL of 0.15 wt% sodium lauryl sulfate and 6g/L NaH₂PO₄, having a pH of about 4.5 at 37°C, with paddles rotating at a speed of 50 rpm.

### BRIEF DESCRIPTION OF THE DRAWINGS

Figure 1 compares the dissolution profile of a solid composite of the present invention with that of a commercial version of tadalafil tablets (Cialis® 20 mg), and with that of tadalafil particles having a particle size of d(0.9)=~50µm and as achieved by conventional means.
Figure 2 compares the dissolution profiles of a solid composite of the present invention with that of micronized tadalafil particles having a particle size of d(0.9)=~4µm.

### DETAILED DESCRIPTION OF THE INVENTION

In one embodiment, the present invention relates to solid composites comprising tadalafil and at least one carrier, wherein at least about 85 wt% of the tadalafil is in a single phase with the at least one carrier.

It is understood that some portion of the tadalafil may not be in the single phase or remain undissolved in the carrier without departing from the scope of the invention. More preferably, at least about 90 wt% of the tadalafil is in a single phase with the at least one carrier. Yet more preferably, at least about 95 wt% of the tadalafil is in a single phase with the at least one carrier. Yet even more preferably, at least about 99 wt% of the tadalafil is in a single phase with the at least one carrier. Most preferably, all or substantially all of the tadalafil is in a single phase with the at least one carrier.

Preferably, at least 85% of the tadalafil is not in crystalline form.

For purposes of the present invention, "tadalafil" means the free base tadalafil and pharmaceutically acceptable salts and solvates thereof, although no intention to limit the scope of tadalafil compounds is intended. Preferably, the tadalafil in the solid composites and in the methods described herein for making the solid composites is the free base.

"Intimate association" or "intimately associated" herein means the at least one carrier and tadalafil interact on the molecular level, there being no easily detectable separate tadalafil phase. That is, the at least one carrier and tadalafil are included in the same single phase.

"Free drug" or "free drug form" herein mean solid particles consisting essentially of tadalafil that are not in intimate association with a carrier.

"Non-crystalline" and "not in crystalline form" mean materials that do not produce X-ray powder diffraction patterns having peaks characteristic of crystalline tadalafil and that do not exhibit a discernable endotherm in differential scanning calorimetry under ordinary conditions - e.g. using heating rates of 2 to 20 degrees per minute. Amorphous tadalafil is an example of non-crystalline tadalafil.

As used herein in connection with a measured quantity, the term "about" refers to the normal variation in that measured quantity that would be expected by the skilled artisan making the measurement and exercising a level of care commensurate with the objective of the measurement and the precision of the measuring equipment used.

Preferably, no crystalline tadalafil is detectable in the solid composites. The tadalafil in the solid composites of the invention are preferably in an amorphous state.

Preferably, the solid composites are solid solutions of tadalafil.

A "solid solution" means a solid, homogenous mixture of at least two components (e.g. tadalafil and a carrier) wherein the components are interspersed on a molecular level.

In solid solutions, the individual physical properties related to the crystalline structure of the components present in lesser amounts, commonly referred to as the solutes, are lost. Presence of the solutes can be detected spectroscopically or by measure of the colligative properties of the solid solution.

Many pharmaceutically acceptable inert solid carriers are suitable for the solid composites of the invention, including sugars and polymers.

Preferably, the carrier is a hydrophilic polymer such as povidone, hydroxypropyl methylcellulose, or polyethylene glycol. Most preferably, the carrier is povidone. Hydroxypropyl methyl cellulose phthalate, polymethyacrylate, and hydroxypropyl cellulose can also be suitable as carriers in the present invention and but not preferred carriers for use in the practice of the present invention.

Preferably, at minimum, one will use at least an amount of carrier sufficient to maintain at least 85% of the tadalafil in intimate association with, preferably in solid solution in, the carrier. Typically, the drug to carrier weight ratio in the solid composites of the present invention is in a range of about 1:0.5 to about 1:20, more preferably about 1:1 to about 1:10, even more preferably about 1:3 to about 1:6 and most preferably about 1:5.

The solid composites of the present invention can be prepared by a) combining tadalafil, at least one carrier, and at least one solvent to form a solution; and b) removing the solvent to obtain the solid composite. Preferably, the solid composite prepared by such a method is in the form of a solid solution, wherein substantially all, most preferably all, of the tadalafil is in solid solution in the at least one carrier.

Optionally, pharmaceutically acceptable excipients may be combined in step a) or added to the solution of step b).

Solvents suitable for preparing the solid composites include organic solvents or a combination of organic solvent(s) with water capable of dissolving at least 85 wt% of the tadalafil and substantially all of the carrier. Preferably, the solvent is capable of dissolving at least 85 wt% of the tadalafil and 85 wt% of the carrier. Even more preferably, the solvent is capable of dissolving substantially all of the tadalafil and carrier. Most preferably, the solvent is capable of dissolving all of the tadalafil and carrier.

Examples of suitable solvents include lower aliphatic alcohols and C₃-C₈ ketones.

"Lower aliphatic alcohols " herein means organic compounds having the general structure R-OH, wherein R is a linear or branched C₁-C₆ alkyl group. Lower aliphatic alcohols that are preferred for use in the process of the invention include methanol, ethanol, isopropyl alcohol (IPA), and butanol. C₃-C₈ ketones useful as solvents in the invention include acetone, methylisobutyl ketone (MIBK) and methylethyl ketone (MEK). Especially preferred solvents are ethanol, acetone, and isopropyl alcohol. Most preferably, the solvent is ethanol. Preferred combinations of solvent and water include acetone and water, preferably in a ratio of acetone: water from 30:1 to 1:1, more preferably in a ratio of from 11:2 to 3:1.

Preferably, the at least one solvent is one in which tadalafil has a solubility of at least about 1.2 mg tadalafil per 1 ml solvent at 25°C.

Preferably, at least 85 wt% of the tadalafil and a majority of the carrier are in solution in the at least one solvent. More preferably, at least 85 wt% of the tadalafil and 85 wt% of the carrier are in solution in the at least one solvent. Even more preferably, substantially all of the tadalafil and carrier are in solution in the at least one solvent. In particularly preferred embodiments, all of the tadalafil and carrier are in solution in the at least one solvent.

In one embodiment of the invention, the combining of tadalafil, at least one carrier and at least one solvent includes the step of mixing a solvent with tadalafil and at least one carrier in any order. The tadalafil, carrier, and solvent are mixed using any suitable mixing method, such as by using magnetic stirrers, mixer stirrers, shakers, or sonification, to mention just a few.

Tadalafil in any form (e.g. crystalline or amorphous) can be used as a starting material to prepare the solid composites.

The tadalafil may be synthesized by any suitable means.

Solvent removal can be by any suitable method. The preferred method is evaporation. Particularly, preferred methods of removing the at least one solvent include fluidized bed drying and spray-drying the solution of tadalafil. "Spray-drying" broadly refers to processes involving breaking up liquid mixtures into small droplets (atomization) and rapidly removing solvent from the mixture. In a typical spray-drying apparatus, there is a strong driving force for evaporation of solvent from the droplets, which may be provided by providing a heated drying gas. Spray-drying processes and equipment are described in Perry's Chemical Engineer's Handbook, pgs. 20-54 to 20-57 (Sixth Edition 1984).

The obtained solid composites of tadalafil in intimate association with the carrier can optionally be recovered and dried.

In a further aspect, the invention provides a solid composite comprising tadalafil and at least one carrier obtainable by the methods defined above.

The solid composites of the invention can be formulated into pharmaceutical compositions. Pharmaceutically acceptable excipients, e.g. surfactants (e.g. sodium lauryl sulfate), binders, fillers, glidants, lubricants, disintegrants and the like, can be used in such compositions. For example, the solid composites can be formulated into solid oral dosage forms such as capsules, tablets, or gelcaps. In one exemplary embodiment, the solid composites are collected and used as is within a capsule or optionally combined with one or more pharmaceutically acceptable excipients, e.g. surfactants (e.g. sodium lauryl sulfate), binders, fillers, glidants, lubricants, disintegrants and the like, in a pharmaceutical composition that can be processed to, e.g., a pharmaceutical dosage form.

Solid composites that are formulated into pharmaceutical compositions and then to a dosage form like a tablet include pharmaceutically acceptable excipients whose functions include helping to bind the active ingredient and other excipients together after compression. Lubricants can be added, for example, during tablet formulation to reduce adhesion and ease release from the tablet punch and dye.
In another embodiment, the solid composites are formulated into pharmaceutical compositions to be administered parenterally, rectally, transdermally, buccally, or nasally. Suitable forms of parenteral administration include an aqueous or non-aqueous solution or emulsion, while for rectal administration suitable forms for administration include suppositories with hydrophilic or hydrophobic vehicle. For topical administration the invention provides suitable transdermal delivery systems known in the art, and for nasal delivery there are provided suitable aerosol delivery systems known in the art. Pharmaceutical compositions to be administered orally are preferred.

As an alternative to collecting the solid composites of tadalafil after solvent removal and then adding one or more excipients, these two steps may be combined in a single step by, for example, spraying the solution containing the tadalafil, carrier and solvent onto to a fluidized bed of the excipient while drying. As a result, the solid composite will be formed on the excipient.

The pharmaceutical compositions may contain additional tadalafil, meaning tadalafil in addition to the tadalafil in the solid composite. Consequently, one can control the rate of dissolution from a composition of tadalafil to match a profile within a large range. For example, where slower rate of dissolution is preferred, the composition would preferably comprise a large percentage of "additional" (free drug) tadalafil as compared to a composition where a higher rate of dissolution is desired.

The tadalafil in free drug form used to adjust the dissolution is preferably that with a particle size distribution such that the d(0.9) value would be greater than about 40µm, for example, about 50µm.

The amount of the solid composite used in a pharmaceutical formulation is preferably an amount that provides a therapeutically effective amount of tadalafil. It will be appreciated that the amount of solid composite used will differ according to the drug:carrier ratio in the particles.

"Effective" or "therapeutically effective" amount of a drug or pharmacologically active agent means an amount of the drug or agent that is nontoxic and sufficient to provide the desired effect, e.g., treatment of erectile dysfunction.

The amount of PDE5 inhibitor administered and the dosing regimen used, will depend on the particular drug selected, the age and general condition of the subject being treated, the severity of the subject's condition, and the judgment of the prescribing physician. Thus, because of patient-to-patient variability, dosages are a guideline only and the physician may adjust doses of the compounds to achieve the level of effective treatment that the physician considers appropriate for the patient. In considering the degree of treatment desired, the physician must balance a variety of factors such as the age of the patient and the presence of other diseases or pre-existing conditions.

The pharmaceutical composition may be administered to a mammal. Preferably, the mammal is a human. Preferably, the pharmaceutical formulation is administered to treat erectile dysfunction.

"Treating," "treated," and "treatment" means at least one of the following: reduction in severity and/or frequency of symptoms, elimination of symptoms and/or underlying cause, prevention of the occurrence of symptoms and/or their underlying cause, or improvement or remediation of damage. For example, the present method of "treating" erectile dysfunction, as the term is used herein, thus encompasses both prevention of the disorder in a predisposed individual and treatment of the disorder in a clinically symptomatic individual.

The solid composites of the invention and pharmaceutical compositions including them preferably allow for the rapid absorption and onset of the PDE inhibitor tadalafil in a mammal.

In particular, tadalafil is preferably released from the solid composite or a pharmaceutical formulation including the solid composite with sufficient solubility for gastro-intestinal absorption in the slightly acidic to neutral pH regions.

Preferably, when tested in an aqueous solution having a pH in the range of from about 4.5 to about 7.0, at least 60% of the tadalafil is released from the solid composite or a pharmaceutical formulation including the composite and dissolves in the solution within about 10 minutes. Preferably, at least 70%, more preferably at least 80%, of the tadalafil is released from the composite or a pharmaceutical formulation including the composite and dissolves in the solution within about 5 minutes under these conditions. Preferably, such dissolution rates are also achieved when composites containing 20mg of tadalafil are tested at conditions at least as stringent as those exemplified by Table 1.

It will be appreciated that, although the solid composites and pharmaceutical formulations comprising the solid composites can be coated (with, e.g., a polymeric coating), generally, when dissolution rates are tested, the solid composites and the pharmaceutical formulations are uncoated.

The compositions of the invention may also include mixtures of tadalafil in the solid composite of the invention together with tadalafil in free drug form. In this case, preferably the particles of the tadalafil in free drug form have a particle size distribution such that the d(0.9) value is greater than 40µm so that intermediate rates of dissolution are achieved. Thus, the skilled artisan will know to vary the ratio of solid composite and tadalafil in free drug form to obtain the desired dissolution rate. Preferably, such a composition would achieve a dissolution rate that matches Cialis® - i.e., preferably, about 50 % of the composition containing 20mg of tadalafil would be dissolved after about 5 minutes and about 70% of the composition would be dissolved after about 10 minutes when a sample of a composition containing 20 mg of tadalafil tested in conditions at least as stringent as those exemplified by Table 1 (e.g. the conditions set forth in Table 1).

### EXAMPLES

A Mini-Glatt model 7069 fluidized bed drier, operated under the following conditions, was used to remove solvent in the Examples described herein:
Preheat inlet: 40°C -60°C
Atomizing press: 1.5-2 bar
Product temperature: 30°C-35°C
Flap (air flow): 0.2 bar
Pump: 9 RPM

Dissolution profiles described herein were determined in a dissolution vessel using a USP Apparatus 2 (Paddles) under the conditions described in Table 1. Samples were analyzed on-line by a UV detector.

**Table 1: Dissolution conditions**

| | |
|---|---|
| Medium | 0.15% aqueous solution sodium lauryl sulfate and 6g/L NaH₂PO₄. pH=~4.5 |
| Volume | 1000 ml |
| Temperature | 37°C |
| Speed | 50 RPM |
| Sampling points: | 5, 10, 20, 30 and 40 minutes |

### Example 1: Tadalafil Solid Solution (Ratio of Active Drug : Carrier 1:5)

A solution was formed by dissolving 300 mg of tadalafil with a particle size d(0.9)=~50µm in 240 ml of ethanol using a sonicator. The term d_{(0.9)} =~50µm refers to the fact that at least 90% (by weight) of the particles have a particle size of less than 50µm.

1500 mg of povidone (PVP K-30) was completely dissolved in the solution using a sonicator. The ethanol was evaporated from the solution using a fluidized bed drier as described above to obtain a dry powder (P-00464).

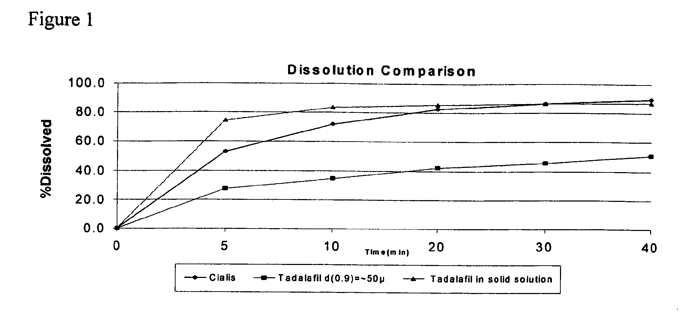

A sample of the dry powder P-00464, containing 20mg Tadalafil was collected and its dissolution profile determined according to the conditions in Table 1. The dissolution profile of P00464 was compared with the dissolution profiles of commercial versions of tadalafil tablets (Cialis® 20 mg) and Active drug substance with a particle size distribution (PSD) such that the value of d_{(0.9)}=^{∼}50µm. The results of dissolution testing are illustrated in Figure 1.

The amount of tadalafil dissolved at the 40 minute time point demonstrates that tadalafil in a solid solution form (composite) has a solubility greater than tadalafil particles with d(0.9)=~50µm. The percent of tadalafil dissolved after 5 minutes demonstrates the greater dissolution rate of tadalafil in a solid solution form, as compared to both tadalafil Cialis® and particles with d(0.9)=~50µm.

### Example 2: Faster Dissolution and Higher Overall Solubility of Tadalafil Solid Solution Compared to Tadalafil with PSD d(0.9)=~4µm

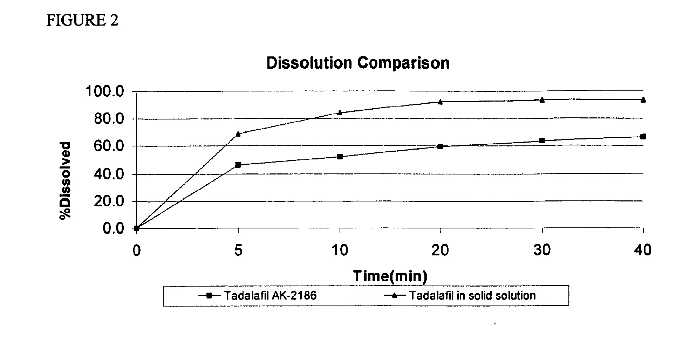

Samples of P-00464 from Example 1 and samples of Tadalafil active material with PSD of d(0.9)=~4µm (designated TAPI AK-2186) were prepared for dissolution testing as described below. The dissolution profiles of the samples were determined according to the conditions in Table 1. The samples were analyzed on-line by UV detection, and were compared. Figure 2 illustrates the averages of the samples for each drug.

### Sample preparation:

### Active drug substance:

1) A 20 mg sample of Tadalafil with PSD of d(0.9)=~4µm (designated TAPI AK-2186) was placed into a glass tube.
2) 5 ml of aqueous 0.05% sodium lauryl sulfate were added to the tube.
3) The sample was placed in a sonicator for 8 minutes, forming a slurry.
4) The slurry was transferred into a dissolution vessel for testing.

### P-00464:

1) A 125 mg (equivalent to 20mg tadalafil) sample of P-00464 from Example 1 was weighed and transferred into a dissolution vessel for testing.

Greater than 90% of the tadalafil in solid solution is dissolved within 40 minutes, while less than 70% of the Tadalafil particles with PSD d(0.9) =~4µm is dissolved at the same point.

As one skilled in the art would appreciate, these results demonstrate that Tadalafil in a solid solution form has a higher dissolution rate and higher solubility than Tadalafil particles with PSD d(0.9) =~4µm, which, in turn, demonstrates that the compositions of the invention have significantly higher solubility as compared with micronized free drug particulate forms of tadalafil.

### Example 3: Tadalafil Solid Solution (Ratio of Active Drug : Carrier-Eudragit L-100 1:0.5)

1000 mg of Tadalafil with particle size d(0.9)=~50µm is dissolved in 1000 ml of ethanol, to form a solution. 500 mg of Eudragit L-100 is dissolved in the solution. Ethanol is evaporated from the solution using a fluidized bed drier to obtain dry powder. The dry powder is collected and may be combined in a pharmaceutical formulation.

### Example 4: Tadalafil Solid Solution (Ratio of Active Drug: Carrier 1:3

300 mg of Tadalafil with particle size d(0.9)=~50µm is dissolved in 240 ml of ethanol using a sonicator, to form a solution. 900 mg of hydroxypropyl methylcellulose is dissolved in the solution using a sonicator. Ethanol is evaporated from the solution using a fluidized bed dryer to obtain dry powder. The dry powder is collected and may be combined in a pharmaceutical formulation.

### Example 5: Tadalafil Solid Solution (Ratio of Active Drug: Carrier 1:7

300 mg of Tadalafil with particle size d(0.9)=~50µm is dissolved in 240 ml of ethanol using a sonicator, to form a solution. 2100 mg of polyethylene glycol is dissolved in the solution using a sonicator. Ethanol is evaporated from the solution using a fluidized bed dryer to obtain dry powder. The dry powder is collected and may be combined in a pharmaceutical formulation.

### Example 6: Tadalafil Solid Solution (Ratio of Active Drug: Carrier 1:20)

300 mg of Tadalafil with particle size d(0.9)=~50µm is dissolved in 240 ml of ethanol using a sonicator, to form a solution. 6000 mg of povidone (PVP k-30) is dissolved in the solution using a sonicator. Ethanol is evaporated from the solution using a fluidized bed dryer to obtain dry powder. The dry powder is collected and may be combined in a pharmaceutical formulation.

### Example 7: Tadalafil Solid Solution (Ratio of Active Drug: Carrier 1:10)

300 mg of Tadalafil with particle size d(0.9)=50~µm is dissolved in 240 ml of ethanol using a sonicator, to form a solution. 3000 mg of povidone (PVP k-30) is dissolved in the solution using a sonicator. Ethanol is evaporated from the solution using a fluidized bed dryer to obtain dry powder. The dry powder is collected and may be combined in a pharmaceutical formulation.

### Example 8: Physical mixture 75% of Tadalafil Solid Solution (Ratio of Active Drug: Carrier 1:5) and 25% of Tadalafil free drug (d(0.9)=~50µm)

90 mg of the powder of example 1 (P-00464 - equivalent to 15mg of Tadalafil) is mixed with 5 mg of Tadalafil Raw material having PSD of d(0.9)=~50µm.

Upon testing this physical mixture using the dissolution medium described above (in example 1), the results at each time interval are near the weighted mean of the dissolution of the two components of the physical mixture (as described in example 1); e.g. dissolution after 5min of mixture of 75% solid solution and 25% free drug is as follows = 0.75*(Dissolution of solid solution at 5min) + 0.25*(Dissolution of free drug at 5min).

### Example 9: Physical mixture 25% of Tadalafil Solid Solution (Ratio of Active Drug: Carrier 1:5) and 75% of Tadalafil free drug (d(0.9)=~50µm)

30 mg of the powder of example 1 (P-00464 - equivalent to 15mg of Tadalafil) mixed with 15 mg of Tadalafil Raw material having PSD of d(0.9)=~50µm.

Upon testing this physical mixture using the dissolution medium described above (example 1), the results at each time interval are near the weighted mean of the dissolution of the two components of the physical mixture (as described in example 1); e.g. dissolution after 5min of mixture of 75% solid solution and 25% free drug is as follows = 0.25*(Dissolution of solid solution at 5min) + 0.75*(Dissolution of free drug at 5min).

## Claims

1. A solid composite comprising tadalafil and at least one carrier wherein at least about 85% by weight of the tadalafil is in a single phase with the at least one carrier.

2. The solid composite of claim 1 wherein the at least about 85 wt% of the tadalafil is not in crystalline form.

3. The solid composite of claim 1 or claim 2 wherein the solid composite is a solid solution.

4. The solid composite of any one of the preceding claims wherein at least 80 wt% of the tadalafil dissolves within about 10 minutes when a sample of solid solution containing about 20 mg of tadalafil is tested in 1000 ml of 0.15% aqueous solution sodium lauryl sulfate and 6g/L NaH₂PO₄, having a pH of about 4.5 at 37°C with paddles rotating at a speed of 50 rpm.

5. The solid composite of claim 4 wherein at least 60 wt% of the tadalafil dissolves within about 5 minutes.

6. The solid composite of claim 4 wherein at least 70 wt% of the tadalafil dissolves within about 5 minutes.

7. The solid composite of any one of the preceding claims wherein at least 99 wt% of the tadalafil is in the single phase.

8. The solid composite of any one of the preceding claims wherein the at least one carrier is a hydrophilic polymer.

9. The solid composite of any one of the preceding claims wherein the at least one carrier is present in an amount such that the ratio of tadalafil to polymer is from about 1:0.5 to about 1:20 by weight.

10. The solid composite of claim 9 wherein the at least one carrier is present in an amount such that the ratio of tadalafil to polymer is from about 1:1 to about 1:10 by weight.

11. The solid composite of claim 10 wherein the at least one carrier is present in an amount such that the ratio of tadalafil to polymer is from about 1:3 to about 1:6 by weight.

12. The solid composite of claim 11 wherein the at least one carrier is present in an amount such that the ratio of tadalafil to polymer is about 1:5 by weight.

13. The solid composite of any one of the preceding claims wherein the carrier is selected from the group consisting of povidone, hydroxypropyl methylcellulose, and polyethylene glycol.

14. A solid composite comprising tadalafil wherein when tested in an aqueous solution having a pH between about 4.5 and 7.0, at least 80 wt% of the tadalafil is released from the solid composite or a pharmaceutical formulation including the composite within about 10 minutes.

15. The solid composite of claim 14 wherein the composite contains at least one carrier.

16. The solid composite of claim 14 or claim 15 wherein at least 60 wt% of the tadalafil is released from the composite or a pharmaceutical formulation including the composite and dissolves in the solution within about 5 minutes.

17. A solid composite comprising 20 mg of tadalafil wherein at least 80 wt% of the tadalafil is released from the solid composite or a pharmaceutical formulation including the composite within about 10 minutes when tested in a medium of 1000 ml of 0.15% aqueous solution sodium lauryl sulfate and 6g/L NaH₂PO₄, having a pH of about 4.5 at 37°C with paddles rotating at a speed of 50 rpm.

18. The solid composite of claim 17 wherein the composite further comprises at least one carrier.

19. The solid composite of claim 17 or claim 18 wherein at least 60 wt% of the tadalafil is released from the composite or a pharmaceutical formulation including the composite and dissolves in the solution within about 5 minutes.

20. A solid composite comprising tadalafil and at least one carrier obtainable by evaporating the solvent from a solution of tadalafil and the at least one carrier.

21. The solid composite of claim 20 wherein the carrier is polyvinylpyrrolidone.

22. The solid composite of claim 21 wherein the tadalafil and polyvinylpyrrolidone are present in a ratio of about 1:5 by weight.

23. A pharmaceutical composition comprising a solid composite as defined in any one of the preceding claims.

24. A pharmaceutical composition of claim 23, further comprising tadalafil in free drug form and having a particle size distribution such that the d(0.9) value is greater than about 40 µm.

25. The pharmaceutical composition of claim 24, wherein 50 wt% of the tadalafil dissolves out of the single phase within about 5 minutes and wherein 70 wt% of the tadalafil dissolves out of the single phase within about 10 minutes when a sample of single phase containing about 20 mg of tadalafil is tested in a medium of 1000 ml of 0.15% aqueous solution sodium lauryl sulfate and 6g/L NaH₂PO₄, having a pH of about 4.5 at 37°C with paddles rotating at a speed of 50 rpm.

26. A method of preparing a solid composite of tadalafil and at least one carrier comprising the steps of:
a) combining tadalafil, at least one carrier, and at least one solvent to form a solution; and
b) removing the solvent to obtain the solid composite.

27. The method of claim 26 further comprising collecting the solid composite and forming a pharmaceutical dosage form.

28. The method of claim 27 wherein pharmaceutically acceptable excipients are combined with the solid composite when forming the pharmaceutical dosage form.

29. The method of claim 27 or claim 28 wherein tadalafil in free drug form and having a particle size distribution such that the d(0.9) value is greater than about 40 µm is combined with the solid composite when forming the pharmaceutical dosage form.

30. The method of any one of claims 26 to 29 wherein the solid composite is a solid solution.

31. The method of any one of claims 26 to 30 wherein the carrier is selected from the group consisting of povidone,, hydroxypropyl methylcellulose, and polyethylene glycol.

32. The method of any one of claims 26 to 31 wherein the solvent is at least one organic solvent or a combination of organic solvent(s) and water.

33. The method of claim 32 wherein the organic solvent is selected from the lower C₁-C₆ aliphatic alcohols and the C₃ - C₈ ketones.

34. The method of claim 33 wherein the solvent is a combination of acetone and water, preferably in a ratio of acetone:water from 30:1 to 1:1, more preferably in a ratio of from 11:2 to 3:1.

35. The method of any one of claims 26 to 34 wherein the solvent is removed by evaporation.

36. The method of any one of claims 26 to 34 wherein the solvent removal is effected with a fluidized bed drier.

37. The method of claim 36 wherein the solution is sprayed into an initially empty fluidized bed drier to remove the solvent.

38. The method of claim 36 or claim 37 wherein the solution is sprayed onto at least one pharmaceutically acceptable excipient in a fluidized bed drier.

39. The method of any one of claims 26 to 34 wherein the solvent removal is effected by spray drying.

40. A tadalafil solid composite obtainable by a process as defined in any one of claims 26 to 39.

41. A tadalafil solid composite having a higher solubility as compared with micronized free drug forms of tadalafil.

42. A tadalafil solid solution having a higher solubility as compared with a free drug form of tadalafil having a particle size distribution such that the d(0.9) value is about 4 µm.

43. A solid oral pharmaceutical composition comprising tadalafil in a non-crystalline form.

44. The solid oral pharmaceutical composition of claim 43 wherein the tadalafil is amorphous.

45. The solid oral pharmaceutical composition of claim 43 or claim 44 wherein at least about 85% of the tadalafil is in a non-crystalline form.
